# EUROPÄISCHE PATENTSCHRIFT

(11) **EP 1 330 307 B1**
(45) Veröffentlichungstag und Bekanntmachung des Hinweises auf die Patenterteilung: **07.09.2005**
(21) Anmeldenummer: 01978431.3
(22) Anmeldetag: 17.10.2001
(51) Int. Cl.: B01J 19/00

(54) **VERFAHREN UND VORRICHTUNG ZUR INTEGRIERTEN SYNTHESE UND ANALYTBESTIMMUNG AN EINEM TRÄGER**
METHOD AND DEVICE FOR THE INTEGRATED SYNTHESIS AND ANALYSIS OF ANALYTES ON A SUPPORT
PROCEDE ET DISPOSITIF POUR EFFECTUER UNE SYNTHESE INTEGREE ET UNE DETERMINATION D'UNE SUBSTANCE A ANALYSER SUR UN SUBSTRAT

(30) Priorität: 17.10.2000 DE 10051396; 17.10.2000 US 240793 P
(43) Veröffentlichungstag der Anmeldung: 30.07.2003
(73) Patentinhaber: Febit AG, 68167 Mannheim (DE)
(72) Erfinder: STÄHLER, Cord, F., 69469 Weinheim (DE); GÜIMIL, Ramon, 69126 Heidelberg (DE); SCHEFFLER, Matthias, 69493 Hirschberg/Leutershausen (DE); STÄHLER, Peer, F., 68169 Mannheim (DE); HEIDBREDE, Anke, 68165 Mannheim (DE)
(74) Vertreter: Tiesmeyer, Johannes
(86) Internationale Anmeldenummer: PCT/EP2001/012027
(87) Internationale Veröffentlichungsnummer: WO 2002/032567

(56) Entgegenhaltungen:
- WO-A-00/13017
- WO-A-00/13018
- WO-A-98/53093
- WO-A-99/60170
- DE-A- 19 910 392

## Beschreibung

Die Erfindung betrifft ein Verfahren und eine Vorrichtung zur integrierten Synthese und Analytbestimmung an einem Träger.

Für die Grundlagenforschung in den Biowissenschaften und für die medizinische Diagnostik sowie für einige andere Disziplinen ist die Erfassung biologisch relevanter Informationen in definiertem Untersuchungsmaterial von herausragender Bedeutung. Dabei liegt die genetische Information in Form einer enormen Vielfalt von unterschiedlichen Nukleinsäuresequenzen vor, der DNA (desoxyribonucleic acid). Die Realisation dieser Information führt über die Herstellung von Abschriften der DNA in RNA (ribonucleic acid) meist zur Synthese von Proteinen, die ihrerseits häufig an biochemischen Reaktionen beteiligt sind.

Zum technischen Hintergrund der Erfindung ist auf die Herstellung und Verwendung von Biochips hinzuweisen. Biochips sind normalerweise miniaturisierte hybride Funktionselemente mit biologischen und technischen Komponenten, z.B. an einer Außenoberfläche oder Innenoberfläche immobilisierte Biomoleküle, die als spezifische Interaktionspartner dienen können. Sie werden für die miniaturisierte, hochparallele Analyse benötigt. Häufig weist die Struktur dieser Funktionselemente Reihen und Spalten auf, man spricht dann von "Arrays". Da Tausende von biologischen bzw. biochemischen Funktionselementen auf einem Biochip angeordnet sein können, müssen diese in der Regel mit mikrotechnischen Methoden angefertigt werden. Konventionelle Biochips haben üblicherweise eine 2D-Geometrie. Davon abweichend kann die Form eines Biochips auch aus der Anordnung von zwei oder mehreren 1D-Strukturen entstehen (z.B. Kapillaren). Eine Weiterführung der Geometrie ist eine 3D-Struktur, bei der die Analyse und ggf. auch Manipulation bzw. Steuerung von Reaktionen in einer 2D-Anordnung erfolgen. Als biologische und biochemische Funktionselemente kommen insbesondere in Frage: DNA, RNA, PNA (bei Nukleinsäuren und ihren chemischen Derivaten können z.B. Einzelstränge, Doppelstränge, Triplex-Strukturen oder Kombinationen hiervon vorliegen), Saccharide, Peptide, Proteine (z.B. Antikörper, Antigene, Rezeptoren), Derivate der kombinatorischen Chemie (z.B. organische Moleküle), Zellbestandteile (z.B. Organellen), Zellen, Mehrzeller, Zellverbänder etc.

Zum technischen Hintergrund und zum Umfeld der vorliegend vorgestellten Erfindung wird auf folgende Patentanmeldungen bzw. Druckschriften verwiesen, deren Inhalt in vorliegender Anmeldung einbezogen wird.
DE 199 40 750 A; WO 0013018 A,
DE 199 40 751 A,
DE 199 40 752 A; WO 0013017 A,
DE 199 57 116; PCT/EP00/01356,
DE 199 35 433.2; PCT/EP00/07445,
DE 199 40 810.6; WO 0012123 A.

In der WO 0013018 A sind Träger für Analytbestimmungsverfahren und Vorrichtungen sowie Verfahren zur integrierten Synthese und Analytbestimmung an solchen Trägern beschrieben. Die Träger dienen als Basis für eine vorzugsweise lichtgesteuerte Synthese einzelner Basen (G, A, C und T) oder ganzer Oligonukleotide (Basensequenzen) zur Bildung einer hochparallelen, -planaren und -dichten Anordnung (Array) dieser Oligonukleotide in einer festen Trägermatrix (Chip). Der Träger enthält eine Struktur aus Mikrokanälen in einem zumindest teilweise durchsichtigen flachen Körper. Die flüssigen Einsatzstoffe werden bei der Rezeptorsynthese oder -immobilisierung durch die Kanäle im Trägerkörper geführt und binden, lokal aktiviert, an den Kanalwänden bzw. an bereits vorher an den Kanalwänden synthetisierten Molekülen. Mit anderen Worten, die Produktion der Chips gemäß der WO 0013018 A bzw. der DE 199 40 750 A umfasst die Herstellung eines vorzugsweise mit Mikrokanälen versehenen Trägerkörpers aus einem geeigneten lichtdurchlässigen Material sowie den biochemischen Beschichtungsvorgang an den Wänden der einzelnen Mikrokanäle, so dass anschließend die polymeren Rezeptoren, z.B. Oligonukleotide, in den Kanälen synthetisiert werden können. Hierbei werden in den einzelnen Kanälen im Träger mittels Fotoaktivierung durch eine geeignete Lichtquelle einzelne Rezeptorbausteine, oligomere Synthone (z.B. Di-, Tri-, Tetra- oder Pentanukleotide) oder ganze Basensequenzen (Oligos) ortsspezifisch angelagert. Dadurch entsteht in jedem Kanal eine Vielzahl an Rezeptor-bestückten Bereichen (spezifische Bindungs- bzw. Hybridisierungsstellen), wobei jeder Bereich aufgrund seiner individuellen Rezeptor-Sequenz-Kombination für die Bindung und anschließende Detektion eines spezifischen Analyten, z.B. eines DNA-Fragments, dient. Die Bereiche sind in einer Dimension des planaren Trägers durch die Wände der Kanäle voneinander getrennt und entlang der einzelnen Kanäle wird zwischen zwei benachbarten Bereichen bei der fotoaktivierten Bindung ein entsprechender Freiraum gelassen. Es entsteht ein hochparalleler, hochintegrierter Array von spezifischen Rezeptoren. Die Synthese der Rezeptoren und spätere Bestimmung von Analyten erfolgt bei dem aus WO 0013018 A bzw. DE 199 40 750 A bekannten System mittels einer Lichtemissions-Detektionseinrichtung. Diese umfasst eine programmierbare Lichtquellenmatrix, eine Detektormatrix sowie den zwischen Lichtquellen- und Detektormatrix vorzusehenden Träger sowie ferner Mittel zur Zufuhr von Fluids in den Träger und zur Ableitung von Fluids aus dem Träger. Zum Stand der Technik betreffend den Aufbau von Lichtemissions-Lichtdetektionseinrichtungen wird auf die WO 0013017 A verwiesen.

Ausgehend von der vorstehend angesprochenen Technologie liegt der Erfindung die Aufgabe zugrunde, ein weiter verbessertes Verfahren sowie eine Vorrichtung zur Synthese. einer Vielzahl von polymeren Rezeptoren zum Zwecke der Bildung eines Rezeptor-Arrays für die Analytbestimmung bereitzustellen.

Zur Lösung der Aufgabe wird unter einem ersten Aspekt ein Verfahren zur integrierten Synthese und Analytbestimmung an einem Träger vorgeschlagen, welches die Schritte umfasst:
a) Bereitstellen eines Trägerkörpers,
b) Leiten einer Flüssigkeit mit Partikeln in oder auf den Trägerkörper,
c) Immobilisieren der Partikel auf mindestens einer inneren oder/und äußeren Oberfläche und/oder in Mikrostrukturen des Trägerkörpers,
d) Leiten einer Flüssigkeit mit darin enthaltenen Rezeptoren oder Bausteinen für die Synthese von polymeren Rezeptoren über die immobilisierten Partikel,
e) orts- oder/und zeitspezifisches Koppeln der Rezeptoren oder Rezeptorbausteine an jeweils vorbestimmten Positionen des Trägerkörpers an die immobilisierten Partikel,
f) gegebenenfalls Wiederholen der Schritte (d) und (e), bis die gewünschten Rezeptoren an den jeweils vorbestimmten Positionen des Trägerkörpers auf den immobilisierten Partikeln synthetisiert worden sind,
g) Inkontaktbringen des Trägers bzw. der synthetisierten Rezeptoren mit einer den oder die zu bestimmenden Analyten enthaltenden Probe, und
h) Bestimmen des oder der Analyten über eine Bindung an die Rezeptoren, die an die immobilisierten Partikel gekoppelt sind.

Bei den Partikeln handelt es sich vorzugsweise um sogenannte Mikrobeads oder Mikrokügelchen (microspheres), an denen eine Funktionalisierung stattfinden kann, die beispielsweise aus Keramik, Glas oder Kunststoff (z.B. auch Polymer-Gel) mit oder ohne Einschlüssen von bzw. im Verbund mit z.B. magnetischen Partikeln hergestellt sein können. Solche Mikrobeads sind erhältlich, z.B. von Duke Scientific, Palo Alto, California 94303, Dynal A.S., N-0212 Oslo, Norway. Allgemein können die Beads unter Standardbedingungen hergestellt werden und erlauben jede Art der Online- bzw. Offline-Oberflächenanalytik für die Qualitätskontrolle.

Mit Hilfe der Partikel (Mikrobeads) sollen betreffende Oberflächenbereiche des Trägerkörpers permanent oder temporär bedeckt werden. Die Anwendung kann z.B. in einer Flusszelle erfolgen. Sie ist aber besonders interessant für geschlossene Mikrostrukturen, deren Mikrokanaloberflächen sich ansonsten jeder Oberflächenanalytik entziehen, was die Qualitätssicherung bei der Oberflächenfunktionalisierung erschwert.

Bei der permanenten Immobilisierung der Beads können diese z.B. kovalent oder nicht kovalent untereinander und/oder mit der Oberfläche des Trägerkörpers verbunden (z.B. verklebt) sein. In der Produktion hat dieses Verfahren den Vorteil, dass das Erzeugen der Oberfläche des Trägerkörpers sehr schnell in wenigen einfachen Verfahrensschritten erfolgen kann. Die oftmals aufwendige Funktionalisierung der Oberfläche für die biochemischen oder molekularbiologischen Anwendungen kann vorher in einem kontinuierlichen oder Batchprozess an der Oberfläche der Beads erfolgen. Hier herrschen zudem optimale fluidische Bedingungen für Mischvörgänge etc.

Der Aufbau einer temporären reaktiven Oberfläche mit Hilfe von Beads in einem Trägerkörper ist besonders interessant. Sie kann im Zusammenhang mit In-situ-Verfahren, wie beispielsweise der In-situ-Synthese und Analyse von DNA/RNA etc. zum Einsatz kommen. Bei der temporären Bead-Oberfläche können Prinzipien wie magnetische Felder, elektrische Felder oder auch eine entsprechende Fluidführung im Trägerkörper als Haltekraftursachen verwendet werden. (Die Beads müssen entsprechend magnetisch oder aber elektrisch geladen sein.) Des Weiteren kann eine chemische oder physikalische Einwirkung (z.B. Reaktion, Diffusion, Licht) dazu benutzt werden, die Fixierung der Partikel zu bewirken. Hierbei können Mikrostrukturen, die mechanische Barrieren darstellen, diesen Effekt unterstützen bzw. ermöglichen. Die Haltekraft muss umso größer sein, je stärker die temporär entstehende Oberfläche aus Bead-Oberflächen später beansprucht wird. Ein großer Vorteil der temporären Immobilisierung liegt in der Kostenreduktion. Von einem Verfahrensdurchgang zum nächsten Verfahrensdurchgang sind lediglich die Beads auszuwechseln und nicht der komplette Trägerkörper. Der Trägerkörper verbleibt beispielsweise als fester Bestandteil im System. Entsprechend komplex kann er gestaltet sein. Dies erlaubt beispielsweise eine Vielzahl an Anschlüssen (z.B. fluidisch, elektrisch, optisch) sowie eine komplexe Fluidführung oder die Integration von Funktionselementen wie Detektions(CCD etc.)- oder Manipulations(Mikrofelder, Mikrotemperierung via CMOS-Schaltung imTrägerkörper)-Komponenten. Im Anschluss an die Anwendung der Mikrobeads zur Schaffung einer reversiblen Oberfläche können diese wieder aus dem Trägerkörper herausgelöst bzw. -gespült werden. Anschließend können die Beads mit z.B. herkömmlichen Methoden weiterverwendet werden, so kann z.B. eine Substanzbibliothek, welche auf den Beads entstanden ist, sehr einfach verfügbar gemacht werden.

Die Grundzüge des Lösungsweges der vorliegenden Erfindung betreffend die Immobilisierung der Partikel am Trägerkörper können wie nachstehend zusammengefasst werden:

Der Trägerkörper / das Trägersystem (z.B. eine Flusszelle, eine Kapillare oder eine Mikrokanalstruktur etc.) wird mit einer Mikrobead-Suspension benetzt, bespült, gefüllt oder durchströmt. Suspensionen geeigneter Konzentration im räumlichen Volumen über der bzw. den betreffenden Trägerkörperoberflächen liefern unter Ausnutzung der Graviation oder elektrischer Felder (bei elektrisch geladenen Beads) bzw. magnetischer Felder (bei magnetischen, insbes. paramagnetischen Beads) oder durch eine entsprechende Fluidführung im Trägerkörper (bei gleichzeitiger Verwendung spezieller fluidischer Systeme) Strukturen, die im Idealfall· einem Monolayer, also einer Einzellage, entsprechen und sich an einer oder mehreren Trägerkörperoberflächen, Membranen, Filtern etc. anlagern.

Diese neue vergrößerte Oberfläche wird von den Beads gebildet, die entsprechend ihrer Anwendung beschichtet bzw. funktionalisiert wurden. Entsprechend ihrer Eigenschaften lassen sich für die mono- bis polyfunktionalen Mikrobeads verschiedene Varianten für die Oberflächenfixierung (Immobilisierung der Beads) unterscheiden. Entscheidend für alle Varianten ist die Ortsgebundenheit während des gesamten Prozesses. Es kann zwischen temporären und permanenten "Beadoberflächen" unterschieden werden.

Permanente "Beadoberflächen":

Die Fixierung der präorganisierten Strukturen (Partikel) kann sowohl kovalent als auch nicht kovalent durch Ausnutzung adhäsiver Kräfte erfolgen. Im Falle des letzteren werden die Beads mit einem lösungsmittelbeständigen Kleber aus der Schmelz-, Kontakt-, Dispersions-, Reaktions-, Polykondensations-, Polymerisations- oder Polyadditionsklebstoffklasse (z.B. einigen Epoxidklebern) auf der betreffenden Oberfläche bzw. den Oberflächen des Trägerkörpers irreversibel fixiert. Das Kleben beruht auf Adhäsion und setzt sich aus drei Komponenten zusammen: der mechanischen (Verankerung und Verzahnungseffekte), der physikalischen (hierzu gehören die klassischen nicht kovalenten Wechselwirkungen, wie z.B. van der Waals-, Dipol-Dipol- und ander elektrostatische Kräfte), und der chemischen Adhäsion (hierzu gehören neben Chemisorption auch kovalente Bindungen zwischen Klebstoff und Trägerkörperoberfläche und/oder Beads).

Die verbleibende bzw. verbleibenden Funktionalitäten, die sich selektiv, zum Teil unter Ausnutzung eines orthogonalen Schutzgruppenkonzeptes, adressieren lassen, dienen dem Ankoppeln von Molekülen bzw. Molekülverbänden. Auf diese Weise lassen sich auf Syntheseplätzen im Biochip (Trägerkörper mit immobilisierten Beads) verschiedene Biopolymere, z.B. Peptide und Oligonukleotide oder Biopolymere mit unterschiedlichen Sequenzen synthetisieren.

Temporäre "Beadoberflächen" / permanent - kovalent:

Bei kovalenter Fixierung der präorganisierten Strukturen (Partikel) werden wiederum zwei Fixierungsvarianten unterschieden: eine vertikale Verknüpfung mit der Trägerkörperoberfläche und eine laterale Verknüpfung zwischen den Mikrobeads.

Für die vertikale Verankerung auf den betreffenden Trägeroberflächen kommen u.a. die klassischen Kopplungsprinzipien der Biopolymersynthese (z.B. Peptid- bzw. Phosphoamiditchemie) in Betracht.

Wählt man für die laterale Verknüpfung ein reversibles Bindungsmotiv, z.B. die redoxpotentialabhängige Bildung eines Disulfides, so gelangt man zu temporären Oberflächen, die sich nach Beendigung des Gesamtprozesses aus dem Trägerkörpersystem rückstandslos entfernen lassen.

Bei mehrfach funktionalen Beadoberflächen dienen die verbleibenden Funktionalitäten dem Ankoppeln von Molekülen bzw. Molekülverbänden. Durch eine geeignete Auswahl oder/und durch Ausnutzung eines orthogonalen Schutzgruppenkonzepts lassen sich die verschiedenen Funktionalitäten selektiv adressieren und auf einem Syntheseplatz im Biochip verschiedene Biopolymere-Beadkonjugate, z.B. Peptid und Oligonukleotid oder Biopolymere mit unterschiedlichen Sequenzen synthetisieren.

Temporäre "Beadoberflächen" / nicht kovalent:

Eine weitere Variante stellt die Fixierung der präorganisierten Strukturen (Bead-Partikel) durch reine Haltekräfte wie Graviationskräfte oder aber elektrische Felder (bei elektrisch geladenen Beads) oder magnetische Felder (bei magnetischen, insbesondere paramagnetischen Beads) oder durch eine entsprechende Fluidführung im Trägerkörper dar.

Neben Mikrostrukturen, die die Fluidführung beeinflussen, ist auch die Fixierung in Mikrostrukturen aufgrund einer Form-, Größen- oder Oberflächenänderung durch chemische, mechanische oder physikalische Einwirkung (z.B. Reaktion, Diffusion, Licht) möglich.

An die so temporär immobilisierten Beads lassen sich nun wiederum Moleküle bzw. Molekülverbände ankoppeln. Durch eine geeignete Auswahl oder/und durch Ausnutzung eines orthogonalen Schutzgruppenkonzepts lassen sich die verschiedenen Funktionalitäten selektiv adressieren und auf einem Syntheseplatz im Biochip verschiedene Biopolymere-Beadkonjugate, z.B. Peptid und Oligonucleotid oder Biopolymere mit unterschiedlichen Sequenzen synthetisieren.

Nach Beendigung des jeweiligen Verfahrensdurchlaufs kann die temporäre Mikrobeadoberfläche wieder aufgelöst und z.B. durch spülen fluidisch entfernt werden. Das Auflösen kann z.B. durch Umkehrung oder auch schnellen Wechsel der magnetischen oder elektrischen Haltefelder oder durch eine Umkehrung der fluidischen Halteströmung erfolgen. Bei der Fixierung durch Form-, Größen- oder Oberflächenänderung ist vor dem Entfernen durch Spülen im Bedarfsfall die Mobilität der Partikel wieder herzustellen.

Mögliche In-situ-Verfahren sind:
- die In-situ-DNA/RNA/LNA/PNA, Peptid- oder Proteinsynthese und Analyse (nasschemisch und/oder fotochemisch),
- farbige Beads (magnetisch oder elektrisch angelagert), z.B. mit Limineszenzanwendung,
- hoch parallele ECL-Anwendungen (ECL-Array),
- vorsynthetisierte Beads, also Beads mit vorsynthetisierten Molekülen, die verlängert werden,
- magnetische Beads mit E-Feld für Hybridisierungsoptimierung (flächig oder pro Kanal oder punktuell/ortsaufgelöst durch integrierten Schaltungsbaustein),
- magnetische Beads mit E-Feld und/oder Temperaturfeld für Hybridisierungsoptimierung,
- Bead-Bead-Interaktionen.

In einer abgewandelten Ausführungsform mit zunächst permanent immobilisierten Partikeln kann es vorgesehen sein, die Partikel auf chemischem Wege von den Trägerwänden zu lösen und schließlich vom Träger durch Spülen etc. zu entfernen.

Wie bereits angedeutet, können bei der besonders bevorzugten "temporären Beadoberfläche" magnetische Felder, elektrische Felder, oder auch eine entsprechende Fluidführung im Trägerkörper zur Erzeugung einer entsprechenden Haltekraft für die Bead-Partikel Anwendung finden. Entsprechend dem verwendeten Halteprinzip müssen die Bead-Partikel magnetisch oder/und elektrisch geladen bzw. polarisiert sein. Beads können beispielsweise einen Eisenoxidkern oder Einschlüsse von magnetischen Partikeln haben. Auf der Oberfläche können diese dann mit Silan beschichtet werden. Solche Mikrobeads werden z.B. als superparamagnetische Partikel mit Abmessungen in der Größenordnung von 1 µm von der Firma Polyscience, Inc., angeboten. Bevorzugt ist die Verwendung von Beads mit mittlerem Durchmesser von 0,2 bis 10 µm, besonders bevorzugt von 0,4 bis 5 µm.

Die mit permanent immobilisierten Beads und die mit temporär immobilisierten Beads präparierten Träger können auf all den Gebieten verwendet werden, die z.B. in der WO 0013018 A bzw. DE 199 40 750 A für Träger mit unmittelbar an den Trägerkörperwänden zu synthetisierenden Rezeptoren beschrieben sind.

Im Rahmen der Erfindung kann es ferner vorgesehen sein, dass die Bead-Partikel mehrfachfunktionalisiert, z.B. bifunktionalisiert, werden, etwa indem in separaten Syntheseschritten an den immobilisierten Bead-Partikeln ein Rezeptoraufbau und das Koppeln bzw. der Aufbau einer rezeptorspezifischen Markierungsgruppe erfolgt.

Ferner kann es im Rahmen der Erfindung vorgesehen sein, dass die Bead-Partikel untereinander wechselwirken (Bead-Bead-Interaktionen).

Allgemein ergibt sich als Anwendungsgebiet die flexible und kostengünstige Darstellung und Auswertung einer großen Zahl von individuellen und spezifischen Messplätzen in miniaturisiertem Format, z.B. durch miniaturisierte, ortsaufgelöste Fotochemie und direkt anschließende Analyse. Dadurch kann in Screening-, Analyse- und Herstellungsverfahren eine großen Messdatenmenge sowie eine Produktvielfalt erzeugt werden als Voraussetzung für die Bewältigung und ggf. Reproduktion der Informationsfülle biologischer Systeme.

Die Anwendungsfelder sind entsprechend vielfältig und umfassen:
- prinzipiell alle Anwendungen von Biochips, insbesondere DNA, RNA und Protein-Arrays,
- Substanzentwicklung und Austesten von entsprechenden Substanzen (u. a. Pharmaforschung, Pharmacogenomics etc.),
- Herstellung bzw. Synthese von biochemischen und molekularbiologischen Substanzen,
- molekulare Diagnostik, In-vitro-Diagnostik (IVD),
- DNA-, RNA- und Protein-Analyse (Genom, Transkriptom, Physiom),
- Screening nach molekularen Interaktionen (Immunologie, Pharmaka, Funktional Genomics),
- Zytologie (u. a. 2D-Zellanalyse analog zu FACS-Technik),
- Molekularbiologie,
- Histologie,
- Forensik.

Die erfindungsgemäße Nutzung von kleinen immobilisierten Trägerpartikeln (Beads) als Basis für die Synthese von Rezeptoren bringt folgende Verbesserungen und Vorteile gegenüber dem Stand der Technik mit sich:
- minimale Kosten für die Festphase (disposable Mikrobeads) und den Trägerkörper, da dieser wiederverwendbar ist;
- einfache und kostengünstige Derivatisierung der Partikeloberfläche im Batch- oder einem kontinuierlichen Verfahren, da die chemischen Modifizierungen vorab außerhalb des Gesamtsystems durchgeführt werden können;
- hohe Funktionsintegration bei niedrigen Verbrauchskosten;
- einfache Qualitätskontrolle, da Standardanalysemethoden verwendet werden können;
- erstmalige Kombination verschiedener Verfahren in einem System (so lässt sich z.B. durch die feste Integration des Mikrofluidmoduls (Trägerkörpers) auch eine aufwendigere Elektronik z.B. zur Temperierung, zur Erzeugung von elektrischen und/oder magnetischen Feldern oder zur Messung bzw. Detektion optischer bzw. elektrischer Signale etc. mit der Mikrofluidik kombinieren. Die Verbrauchsmaterialskosten bleiben dennoch vergleichsweise gering.
- Es wird eine systematische Kontrolle und Qualitätssicherung der reaktiven Oberflächen (Bead-Oberflächen) in der Produktion und ferner die Verwendung von Mikrofluidik in geschlossenen Mikrostrukturen bei den Synthese- und Analyseschritten möglich,
- über eine Messung z.B. der Eigenfluoreszenz der Beads oder aber angelagerter Fluoreszenzmarker oder ggf. anderer Label kann die Dichte der Beads vor dem Verfahren festgestellt werden. Diese Label können gemäß einer Verfahrensvariante bei Bedarf im Vorfeld des eigentlichen Verfahrens auch wieder entfernt werden.

Weiterbildungen des Verfahrens zur integrierten Synthese und Analytbestimmung nach Anspruch 1 sind in den Ansprüchen 2 - 15 angegeben.

Gegenstand der Erfindung ist gemäß Anspruch 16 auch ein Verfahren zur Herstellung von rezeptorbeschichteten Partikeln für die Bestimmung von Analyten umfassend die Schritte:
a) Bereitstellen eines Trägerkörpers,
b) Leiten einer Flüssigkeit mit Partikeln in oder auf den Trägerkörper,
c) Immobilisieren der Partikel auf mindestens einer inneren oder/und äußeren Oberfläche des Trägerkörpers,
d) Leiten einer Flüssigkeit mit darin enthaltenen Rezeptoren oder Bausteinen für die Synthese von polymeren Rezeptoren über die immobilisierten Partikel,
e) orts- oder/und zeitspezifisches Koppeln der Rezeptoren oder Rezeptorbausteine an jeweils vorbestimmten Positionen des Trägerkörpers an die immobilisierten Partikel,
f) gegebenenfalls Wiederholen der Schritte d) und e), bis die gewünschten Rezeptoren an den jeweils vorbestimmten Positionen des Trägerkörpers auf den immobilisierten Partikeln synthetisiert worden sind.

Die mit Rezeptoren versehenen Partikel können beispielsweise aus dem Trägerkörper ausgebracht und später an anderer Stelle, beispielsweise in einem anderen Trägerkörper, für die Bestimmung von Analyten herangezogen werden.

Gegenstand der Erfindung ist gemäß Anspruch 17 auch ein Verfahren zum Bestimmen eines oder mehrerer Analyten auf einem Träger umfassend die Schritte:
a) Bereitstellen eines Trägerkörpers mit mindestens einer inneren oder/und äußeren Oberfläche, wobei Partikel mit daran gekoppelten Rezeptoren an jeweils vorbestimmten Positionen der Oberfläche immobilisiert sind, wobei Partikel in unterschiedlichen Bereichen unterschiedliche Rezeptoren tragen,
g) Inkontaktbringen des Trägerkörpers mit einer den oder die zu bestimmenden Analyten enthaltenden Probe, und
h) Bestimmen des oder der Analyten über eine Bindung an die Rezeptoren, die an die immobilisierten Partikel gekoppelt sind.

Gegenstand der Erfindung ist gemäß Anspruch 18 auch ein Verfahren zum Bestimmen eines oder mehrerer Analyten auf einem Träger umfassend die Schritte:
a) Bereitstellen eines Trägerkörpers mit mindestens einer inneren oder/und äußeren Oberfläche, wobei Partikel mit daran gekoppelten Rezeptoren an der Oberfläche immobilisiert sind, wobei mehrere kodierte Partikelspezies mit jeweils unterschiedlichen Rezeptoren verwendet werden und die Immobilisierung eine magnetische oder/und elektrische Wechselwirkung umfasst,
g) Inkontaktbringen des Trägerkörpers bzw. der Rezeptoren mit einer den oder die zu bestimmenden Analyten enthaltenden Probe, und
h) Bestimmen des oder der Analyten über eine Bindung an die Rezeptoren, die an die immobilisierten Partikel gekoppelt sind.

Die Codierung der Partikelspezies kann z.B. eine Farbcodierung, eine Größencodierung oder/und eine Codierung in Form einer rezeptorspezifischen Markierungsgruppe an der Partikeloberfläche sein.

Gegenstand der Erfindung ist gemäß Anspruch 19 auch eine Vorrichtung zur Durchführung des Verfahrens nach einem der Ansprüche 1 - 15 oder zur Durchführung wenigstens eines der Verfahren nach den Ansprüchen 16, 17 oder 18, mit einem Trägerkörper, welcher wenigstens einen inneren oder/und einen äußeren Oberflächenbereich zur Anlagerung von an ihrer Oberfläche funktionalisierten Partikeln, insbesondere Mikrobeads, aufweist, Mitteln zur Zufuhr der Partikel zu dem wenigstens einen Oberflächenbereich des Trägerkörpers, einer Einrichtung zur Immobilisierung der Partikel an den wenigstens einen Oberflächenbereich des Trägerkörpers, Mitteln zur Zuführung von Rezeptoren oder Rezeptorbausteinen A, B zu auf dem wenigstens einen Oberflächenbereich immobilisierten Partikeln und Mitteln zur Steuerung des orts- und/oder zeitspezifischen Koppelns der Rezeptoren oder Rezeptorbausteine A, B an die immobilisierten Partikel an jeweils vorbestimmten Positionen des Trägerkörpers.

Weiterbildungen der erfindungsgemäßen Vorrichtung sind in den Ansprüchen 20 - 30 angegeben.

In einer erweiterten Darstellung können Aspekte der Erfindung wie folgt umschrieben werden: Sie betrifft ein Verfahren zur In-situ-Herstellung von permanenten oder temporären Oberflächen, bestehend aus einer Vielzahl in Abhängigkeit zur geplanten Anwendung derivatisierten Partikeln (Beads) geeigneter Dimension (Durchmesser im Bereich von µm oder nm).

In Ausgestaltung des Verfahrens kommt als Trägersystem z.B. eine Flusszelle, eine Kapillare oder eine Mikrokanalstruktur, etwa aus Gläsern, Platten aus unterschiedlichen Materialien, wie Si, Ge oder aber Kunststoffen, in Frage. Ferner können als Trägersysteme Membranen, Filter etc. in Frage kommen. Die Beads werden auf dem Trägersystem präorganisiert.

Die Präorganisation kann z.B. durch Gravitation erfolgen, wobei es zur Ausbildung von Strukturen kommt, die vorzugsweise einem Monolayer oder einem geordneten Multilayer entsprechen.

Einerseits kann die Präorganisation durch elektrische Felder (bei elektrisch geladenen Beads) erfolgen, wobei es zur Ausbildung von Strukturen kommt, die vorzugsweise einem Monolayer oder einem geordneten Multilayer entsprechen.

Andererseits kann die Präorganisation durch magnetische Felder (bei paramagnetischen Beads) erfolgen, wobei es zur Ausbildung von Strukturen kommt, die vorzugsweise einem Monolayer oder einem geordneten Multilayer entsprechen.

Andererseits kann die Präorganisation durch eine entsprechende Fluidführung im Trägerkörper (bei gleichzeitiger Verwendung spezieller fluidischer Systeme) erfolgen, wodurch es zur Ausbildung von Strukturen kommt, die vorzugsweise einem Monolayer oder einem geordneten Multilayer entsprechen.

Zweckmäßigerweise werden die Beads aus einer Suspension geeigneter Konzentration im räumlichen Volumen über der bzw, den Oberflächenbereichen des Trägerkörpers an einem oder mehreren Oberflächenbereichen angelagert.

Die temporäre Oberfläche der präorganisierten Beads wird durch Haltekräfte, wie Gravitationskräfte oder aber elektrische Felder (bei elektrisch geladenen Beads) oder magnetische Felder (bei magnetischen Beads) oder durch eine entsprechende Fluidführung im Trägerkörper fixiert und der geplanten Anwendung zugänglich gemacht.

In anderer Ausgestaltung können die präorganisierten Beads lateral kovalent miteinander verwendet werden und so im Träger fixiert werden, dass sie der geplanten Anwendung zugänglich gemacht werden.

In Abhängigkeit des Bindungsmotivs erhält man eine temporäre oder permanente Oberfläche.

Durch Verwendung eines reversiblen Bindungsmotivs (z.B. die redoxaktive Thiolchemie) erhält man eine temporär fixierte Oberfläche.

In weiterer Ausgestaltung werden die präorganisierten Beads vertikal kovalent mit der Trägerkörperoberfläche verknüpft und so im Trägerkörper fixiert, dass sie der geplanten Anwendung zugänglich gemacht werden.

Für die vertikale Verankerung auf den Trägerkörperoberflächen kommen u.a. die klassischen Kopplungsprinzipien der Biopolymersynthese (z.B. Peptid- bzw. Phosphoamiditchemie) in Betracht.

In anderer Ausgestaltung werden die präorganisierten Beads durch Adhäsion auf den Trägerkörperoberflächen verklebt und so im Trägerkörper fixiert, dass sie der geplanten Anwendung zugänglich gemacht werden.

Zum Verkleben kommen folgende Klebstoffklassen in Betracht: Schmelz-, Kontakt-, Dispersions-, Reakitions-, Polykondensations-, Polymerisations- oder Polyadditionsklebstoffe. Insbesondere eignet sich zum Verkleben Polyadditionsklebstoff auf lösungsmittelstabiler Epoxidbasis.

Die verbleibenden Funktionalitäten dienen für das Ankoppeln der in Betracht kommenden Moleküle bzw. Molekülverbände.

Die multifunktionalen Beadoberflächen dienen vorzugsweise zum Ankoppeln verschiedener Spezies, so dass auf einem Syntheseplatz im Biochip (Trägerkörper mit immobilisierten Beads) verschiedene Biopolymere-Beadkonjugate z.B. Peptid oder Oligonucleotid oder Biopolymere mit unterschiedlichen Sequenzen synthetisieren kann. Die skann durch Ausnutzung eines orthogonalen Schutzgruppenkonzepts, mit dem sich die verschiedenen Funktionalitäten selektiv adressieren, erfolgen.

Die Erfindung ist zu Herstellung bzw. Analyse von Substanzbibliotheken geeignet. Durch Leiten einer Probe über den Träger mit den immobilisierten Beads können Bestandteile der Probe über spezifische Bindungsereignisse extrahiert werden. Dabei können insbesondere Wechselwirkungen zwischen Biornolekülen wie Proteinen oder Peptiden und den Beads untersucht werden, so dass die Erfindung auch für das Feld der Proteomanalyse (Proteomics) geeignet ist.

Die Erfindung wird im Folgenden anhand der Figuren näher erläutert.
- Fig. 1: zeigt in einer Blockschaltbild-Darstellung eine Vorrichtung nach der Erfindung.
- Fig. 2a und 2b: zeigen ausschnittsweise Längsschnittdarstellungen eines Mikrokanals mit darin befindlichen Bead-Partikeln im nicht-immobilisierten Zustand (Fig. 2a) und im immobilisierten Zustand (Fig. 2b).
- Fig. 3a - 3c: zeigen in ausschnittsweisen Längsschnittdarstellungen entsprechend der Perspektive in den Figuren 2a und 2b einen Fluidkanal des Trägerkörpers während dreier verschiedener Schritte bei der Durchführung eines Syntheseverfahrens.
- Fig. 4a: zeigt in einer Draufsicht einen Schnitt durch einen Fluidkanal des Trägerkörpers, wobei ein auf den Querschnitt projiziertes Belichtungsmuster einer Belichtungsmatrix mit eingezeichnet worden ist.
- Fig. 4b: zeigt einen vergrößerten Ausschnitt aus Fig. 4a.
- Fig. 5: zeigt in einer Blockschaltbild-Darstellung eine Variante der Vorrichtung nach der Erfindung.
- Fig. 6: zeigt in einer Blockschaltbild-Darstellung eine weitere Variante der Vorrichtung nach der Erfindung.
- Fig. 7: zeigt ein Spulenpaar aus Fig. 6 in der in Fig. 6 mit dem Pfeil VII angedeuteten Blickrichtung.
- Fig. 8: zeigt in einer ausschnittsweisen Längsschnittdarstellung einen Fluidkanal des Trägers mit einer Porenwand zur lmmobilisierung von Bead-Partikeln.
- Fig. 9: zeigt ausschnittsweise eine Draufsicht auf die Porenwand aus Fig. 8.
- Fig. 10: zeigt eine ausschnittsweise Draufsicht auf eine Porenwand mit einer dichteren Porenverteilung als in Fig. 9, wobei die Poren in Fig. 10 mehr oder weniger ungeordnet verteilt sind.
- Fig. 11: zeigt ein weiteres Beispiel einer mit Poren versehenen Wand.
- Fig. 12: zeigt eine ausschnittsweise Längsschnittdarstellung eines Fluidkanals des Trägerkörpers mit einer porösen Zwischenwand, welche ein Array aus Säulen und Vertiefungen bildet.
- Fig. 13: zeigt eine ausschnittsweise Draufsicht auf die Zwischenwand aus Fig. 12.
- Fig. 14: zeigt in einer ausschnittsweisen Längsschnittdarstellung eine Variante einer bereichsweise porösen Zwischenwand der in Fig. 12 gezeigten Art.
- Die Figuren 15 - 17: zeigen in stark vergrößerten Ausschnitten und in Draufsichtsdarstellungen Formen und Verläufe verschiedener Varianten von Mikrokanälen in einem Trägerkörper.

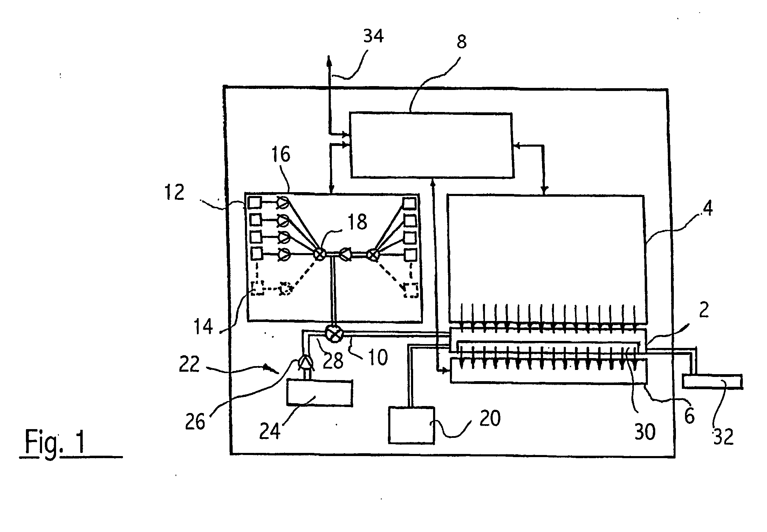

Fig. 1 zeigt eine schematische Blockdarstellung einer erfindungsgemäßen Vorrichtung für ein lichtgestütztes, integriertes Synthese- und ggf. Analyseverfahren. Die Vorrichtung umfasst einen Trägerkörper 2, der von in Fig. 1 nicht erkennbaren Kanälen für den Fluidtransport durchsetzt ist. Der Trägerkörper 2 kann in einer Weise ausgebildet sein, wie es z.B. in der WO 0013018A oder in der deutschen Patentanmeldung DE 199 35 433.2 und der korrespondierenden internationalen Patentanmeldung PCT/EP00/07445 beschrieben ist. Deren Offenbarungsgehalt wird insoweit in die vorliegende Anmeldung einbezogen.

Der Trägerkörper 2 befindet sich zwischen einer zur Erzeugung bestimmter Belichtungsmuster programmierbar steuerbaren Belichtungsmatrix 4 und einer Lichtdetektormatrix 6. Für Licht der Belichtungsmatrix 6 ist der Trägerkörper 2 transparent.

Bei der Belichtungsmatrix kann es sich z.B. um eine Lichtquellenmatrix handeln, die ein zweidimensionales Array aus Leuchtdioden, insbesondere UV-Dioden, oder Laserelementen, insbesondere UV-Laserelementen, umfasst. Als Belichtungsmatrix kommt auch eine Matrix aus steuerbaren Reflexionselementen in Frage, wobei dann eine zusätzliche Lichtquelle erforderlich ist. Als Belichtungsmatrix kommt ferner eine Lichtventilmatrix, etwa eine LCD-Matrix, in Frage. Die auch im Zusammenhang mit der vorliegenden Erfindung bevorzugten Möglichkeiten der Realisierung einer Belichtungsmatrix sind in der WO 0013017 und in der DE 199 40 752 A näher beschrieben.

Die Programmierbarkeit der Belichtungsmatrix 4 ist in die Systemkomponente 8 integriert, welche einen Steuerrechner umfasst, der entsprechende Steuersignale an die Belichtungsmatrix 4 abgibt. Die Belichtungsmatrix 4 belichtet den transparenten Träger 2 nach Maßgabe des jeweiligen vom Steuerrechner 8 im aktuellen Belichtungsschritt vorgegebenen Belichtungsmusters. Über ein fluidisches Anschlusssystem 10 werden die vom Fluidikmodul 12 bereitgestellten Fluide in den Trägerkörper 2 transportiert und in dessen in der Zeichnung nicht dargestellten Mikrokanalstruktur an die Reaktionsbereiche für die Rezeptorsynthese weitergeleitet.

Das in den Trägerkörper 2 eintretende Licht kann ggf. für Absorptionsmessungen, für die Aktivierung von Fotoreaktionen oder/und für das Anregen von Fluoreszenz genutzt werden. Das aus dem Trägerkörper 2 austretende Licht kann beispielsweise das im Durchlicht den Trägerkörper 2 passierende Licht der Belichtungsmatrix 4 sein. Es kann sich dabei jedoch auch um Lichtsignale handeln, die in den einzelnen Reaktionsbereichen in dem Trägekörper 2 durch beispielsweise Fluoreszenz oder Lumineszenz erzeugt werden. Die Detektormatrix 6, die zum Beispiel aus einem CCD-Chip mit oder ohne zwischengeschalteten optischen Elementen besteht, ist in Fig. 1 so gegenüber der Belichtungsmatrix 4 mit dem dazwischen liegenden Trägerkörper 2 angeordnet, dass dadurch eine dreifache Matrixanordnung aus Belichtungs-, Träger- und Detektormatrix entsteht. Das Fluidmodul 12 dient der Versorgung von Reaktionsbereichen im Trägerkörper 2 zum Beispiel mit Einsatzstoffen, Schutzgasen, Chemikalien, wie Lösemitteln, etc. und Probenmaterial. Das Fluidmodul 12 besteht aus Tanks 14, die durch Pumpen 16 und Ventile 18 in geeigneter Weise entleert werden. Die Tanks 14 können einzeln oder im Cluster ausgewechselt oder neu gefüllt werden. Permanent benötigte Fluide, wie beispielsweise Schutzgas, können auch mittels Leitungen von außen liegenden Reservoirs zugeführt werden.

Der fluidische Abfall der verschiedenen Verfahrensschritte kann in einem Abfallsystem 20 aufgefangen werden.

Die Vorrichtung umfasst ferner Mittel 22 zur Zuführung von oberflächenfunktionalisierten Partikeln, vorzugsweise Mikrobeads, zu dem Trägerkörper 2. Die Mittel 22 umfassen ein Reservoir 24 für die Bereitstellung einer Partikelsuspension (Mikrobead-Suspension) sowie eine Pumpe 26 zur Förderung der Suspension aus dem Reservoir 24 in das Kanalsystem des Trägerkörpers 2. Der Trägerkörper 2 ist über eine Leitung 28 mit der Pumpe 26 verbunden. Die Mittel 22 zur Zuführung oberflächenfunktionalisierter Partikel zum Trägerkörper 2 können alternativ in das Fluidmodul 12 integriert sein.

Nur allgemein schematisch ist in Fig. 1 bei 30 eine Einrichtung zur temporären Immobilisierung der aus dem Reservoir 24 zugeführten Partikel an Oberflächen der Fluidkanäle des Trägerkörpers 2 angedeutet.

Mit 32 ist in Fig. 1 ein Auffangsystem für Partikel mit an der Oberfläche ansynthetisierten Rezeptoren bezeichnet.

Der Steuerrechner 8 übernimmt die Steuerung bzw. Regelung des Systems. Hierunter fallen auf der Basis der Berechnung der Sonden- bzw. Rezeptorsequenzen für die einzelnen Reaktionsbereiche die Steuerung der Belichtungsmatrix 4 sowie des Fluidmoduls 12. Hierunter fallen ferner die Steuerung der Einrichtung 30 zur temporären Immobilisierung der Partikel (Mikrobeads) an den Fluidkanaloberflächen im Trägerkörper 2. Ferner erfasst der Steuerrechner 8 die Daten der Detektormatrix 6, um diese auszuwerten oder ggf. zur Auswertung über eine Schnittstelle 34 an daran anzuschließende System weiterzugeben.

Zur Durchführung des erfindungsgemäßen Verfahrens zur integrierten Synthese und Analytbestimmung an dem Träger 2 wird in einem ersten Schritt die Pumpe 26 aktiviert, damit sie aus dem Reservoir 24 die Mikrobead-Suspension in das Kanalsystem des Trägerkörpers 2 fördert. Der Steuerrechner 8 aktiviert die Einrichtung 30, woraufhin sich Mikrobeads aus der Suspension an Fluidkanalflächen des Trägerkörpers 2 absetzen und dort verbleiben. Der Vorgang kann mittels der Detektormatrix 6 optisch überwacht werden.

Nach Immobilisierung einer Lage von Mikrobeads im Trägerkörper 2 und nach Ausspülen der für das weitere Verfahren nicht mehr benötigten Restsuspension können die Rezeptorsyntheseschritte erfolgen. Hierzu steuert der Steuerrechner 8 jeweils die Belichtungsmatrix 4 nach einem Programm zur Erzeugung der betreffenden Belichtungsmuster an. An den belichteten Stellen kommt es zur Abtrennung fotolabiler Schutzgruppen von den Mikrobeads. An diesen entschützten Stellen können dann Rezeptorbausteine ankoppeln, die mit einem Fluid aus dem Fluidmodul 12 zugeführt werden und ihrerseits fotolabile Schutzgruppen tragen, die bei einem nächsten oder späteren Syntheseverfahrensschritt durch Belichtung abgetrennt werden können, um einen nächsten Synthesebaustein an die betreffenden entschützten Stellen ankoppeln zu können. Die lichtgesteuerte Synthese von Rezeptoren (allerdings unmittelbar auf Flächen des Trägerkörpers) ist insoweit in der WO 0013018 A und der DE 199 40 750 A beschrieben.

Nachdem die gewünschten Rezeptoren an den jeweils vorbestimmten Positionen des Trägerkörpers 2 auf den immobilisierten Mikrobeads synthetisiert worden sind, kann der Schritt der Analytbestimmung erfolgen, wobei die Rezeptoren mit einer den oder die zu bestimmenden Analyten enthaltenden Probe in Kontakt gebracht werden, wobei dann das Bestimmen des oder der Analyten über eine Bindung an die Rezeptoren, d.h. über den Nachweis der Bindung an bestimmte Rezeptoren erfolgt.

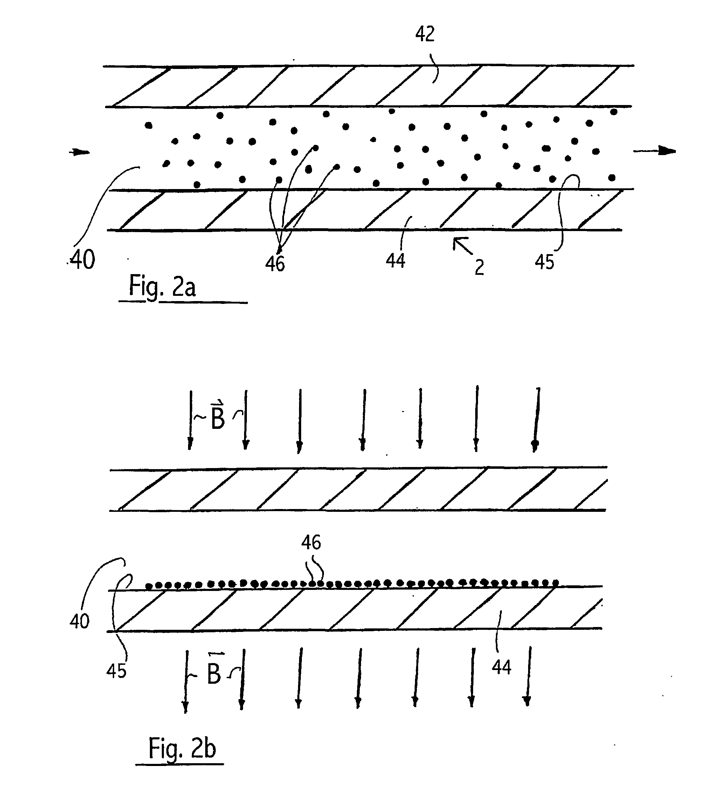

Vorzugsweise umfasst die Einrichtung 30 zur Immobilisierung der Mikrobeads Mittel zur Erzeugung eines Magnetfeldes (B-Feldes) quer zur Strömungsrichtung in den Fluidkanälen des Trägerkörpers 2. Die Mikrobeads sind in diesem Fall magnetisch präpariert. Damit lässt sich dann die anhand der Figuren 2a und 2b zu erläuternde Situation der Immobilisierung der Mikrobeads 46 herbeiführen. Fig. 2a zeigt in einer Ausschnittsdarstellung einen Längsschnitt eines Fluidkanals (Mikrokanals) 40 eines Trägerkörpers 2 mit einer transparenten Deckschicht 42 und einem vorzugsweise ebenfalls transparenten Boden 44. Der Kanal 40 wird in der Darstellung gemäß Fig. 2a von einer Mikrobead-Suspension mit magnetischen Mikrobeads 46 durchströmt.

Nach Einschalten des B-Feldes kommt es zur Situation gemäß Fig. 2b, in der die Beads 46 aufgrund ihrer magnetischen Wechselwirkung mit dem B-Feld an der inneren Oberfläche des Mikrokanals 40 festgehalten und somit immobilisiert worden sind. Zur Erzeugung des B-Feldes können Permanentmagneten oder/und elektrische Magnetspulen herangezogen werden.

Die Situation gemäß Fig. 2b kann auch herbeigeführt werden, wenn elektrisch geladene bzw. elektrisch polarisierte Mikrobeads verwendet werden und zu deren Immobilisierung in den Fluidkanälen 40 des betreffenden Trägerkörpers 2 ein elektrisches Feld quer zur Strömungsrichtung erzeugt wird. Dabei kann es vorgesehen sein, dass ein ausgedehntes elektrisches Feld über die Fläche des Trägerkörpers hinweg erzeugt wird. Andererseits kann es auch vorgesehen sein, dass punktuell und ortsaufgelöst in Bezug auf bestimmte Reaktionsbereiche elektrische Felder erzeugt werden, z.B. mittels in den Trägerkörper integrierten elektronischen Elementen, wie etwa CMOS-Transistoren oder ggf. transparente Elektroden, wie sie bei Flüssigkristallanzeigen verwendet werden.

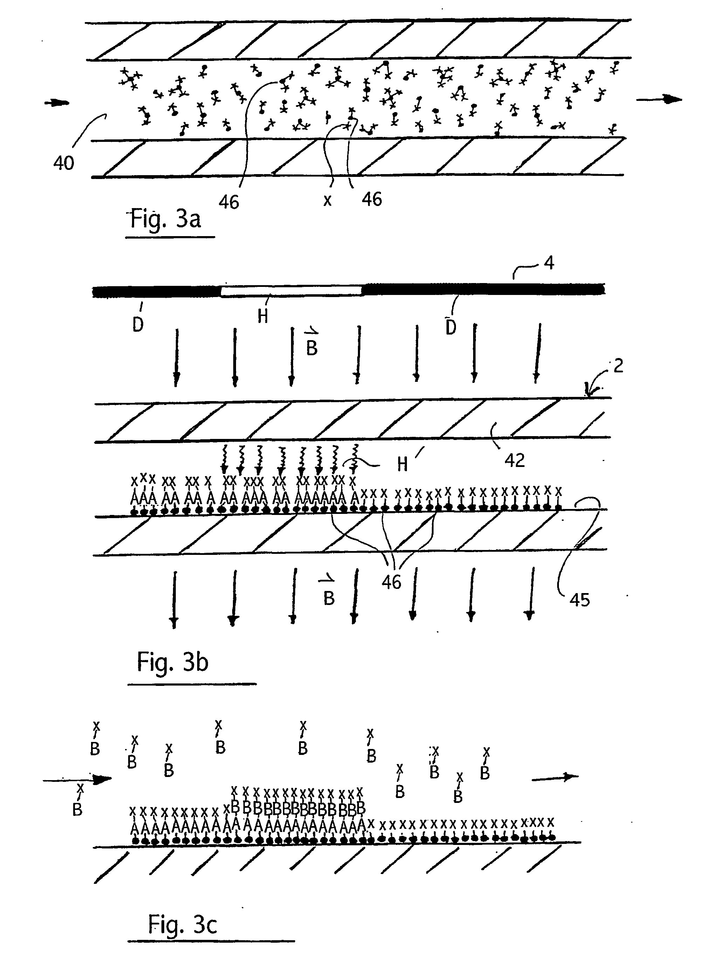

Fig. 3a zeigt in einer der Fig. 2a entsprechenden Darstellung den Mikrokanal 40, der von magnetischen Mikrobeads mit fotolabilen Schutzgruppen x in einer betreffenden Suspension durchströmt wird.

Fig. 3b illustriert einen Syntheseschritt, nachdem durch Einschalten des B-Feldes magnetische Mikrobeads 46 in einer im Beispielsfall planar angeordneten 1-Layerschicht immobilisiert worden sind und nachdem in einem vorausgegangenen Syntheseschritt einige Mikrobeads bereits einen kovalent angekoppelten Synthesebaustein A mit fotolabiler Schutzgruppe x aufweisen. 4 kennzeichnet in Fig. 3b die Belichtungsmatrix, wobei sie in dem betrachteten aktuellen Belichtungsschritt unterhalb der Stelle H den Trägerkörper 2 durch dessen transparente Deckschicht 42 hindurch belichtet, wohingegen von den Stellen D der Belichtungsmatrix 4 momentan kein Licht zum Trägerkörper 2 hin emittiert oder ggf. reflektiert wird, so dass unterhalb von D ein "Dunkelfeld" vorliegt. Die Strahlung des "Hellfeldes" H führt in dem belichteten Bereich zur Abspaltung der fotolabilen Schutzgruppen x, so dass an den so entschützten Mikrobeads 46 bzw. Synthesebausteinen A weitere Synthesebausteine B mit fotolabilen Schutzgruppen x ankoppeln können, wie dies in Fig. 3c angedeutet ist. Die Belichtungsschritte mit programmiert gesteuerter Auswahl des jeweiligen Belichtungsmusters und die fluidische Zufuhr betreffender Synthesebausteine mit fotolabilen Schutzgruppen wird wiederholt, bis die gewünschten Rezeptoren an den jeweils vorbestimmten Positionen des Trägerkörpers 2 auf den immobilisierten Partikeln 46 synthetisiert worden sind.

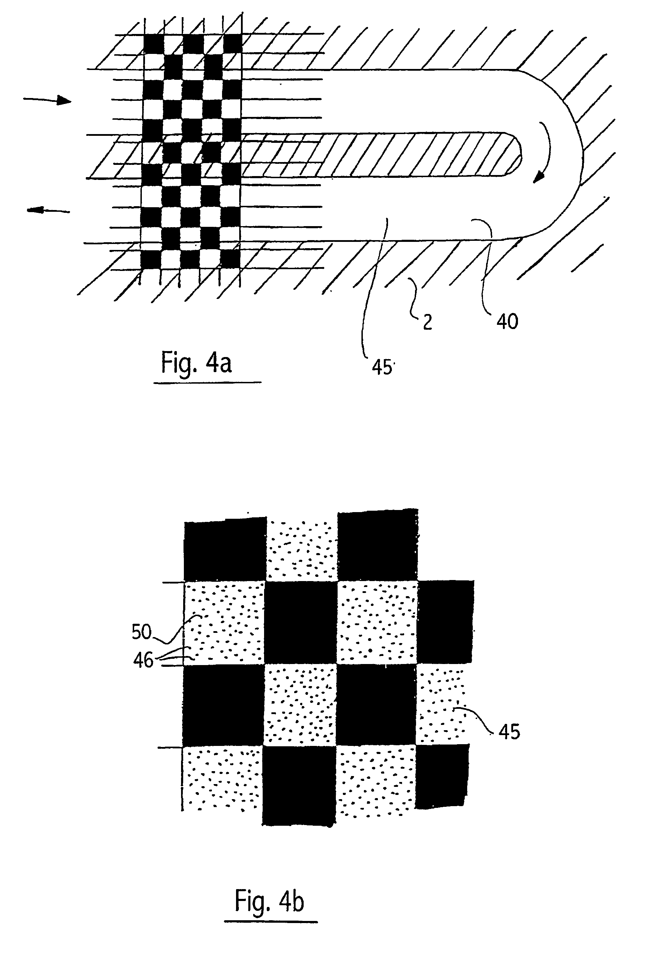

Fig. 4a zeigt in einer ausschnittsweisen Draufsicht auf einen Schnitt durch den Trägerkörper 2 einen Fluidkanal (Mikrokanal) 40 und einen Ausschnitt aus einem Schachbrettmuster-artigen Hell-dunkel-Lichtfeld, wie es von der Belichtungsmatrix 4 erzeugt werden kann.

Fig. 4b zeigt einen vergrößerten Ausschnitt aus Fig. 4a, nämlich einen Bereich des Fluidkanalbodens mit dem darauf projizierten Hell-dunkel-Lichtfeld. Darin ist zu sehen, dass in einem jeweiligen Belichtungsfeldelement eine größere Anzahl an immobilisierten Mikrobeads 46 vorgesehen sein kann. Die Dichte der Belegung kann z.B. durch die Konzentration der Mikrobeads 46 in der ursprünglichen Mikrobead-Suspension oder durch entsprechende Wahl der Zeit, in der die Fluidkanäle 40 bei eingeschaltetem Magnetfeld (oder ggf. E-Feld) von der Mikrobead-Suspension durchströmt werden. Wie bereits erwähnt, kann die Belegung der Kanäle 40 mit immobilisierten Beads 46 großflächig, z.B. durch ein entsprechendes B-Feld oder ggf. E-Feld, erfolgen. Alternativ könnte die Belegung auch ortsaufgelöst, z.B. durch ortsaufgelöste lokale E-Felder, B-Felder oder ggf. auch durch optische Lichtfallen (analog einer sogenannten Laserpinzette) erfolgen.

In Bezug auf die Figuren 4a und 4b ist darauf hinzuweisen, dass für einen folgenden Belichtungsschritt ein anderes Belichtungsmuster als das gezeigte Schachbrett-Muster erzeugt werden kann.

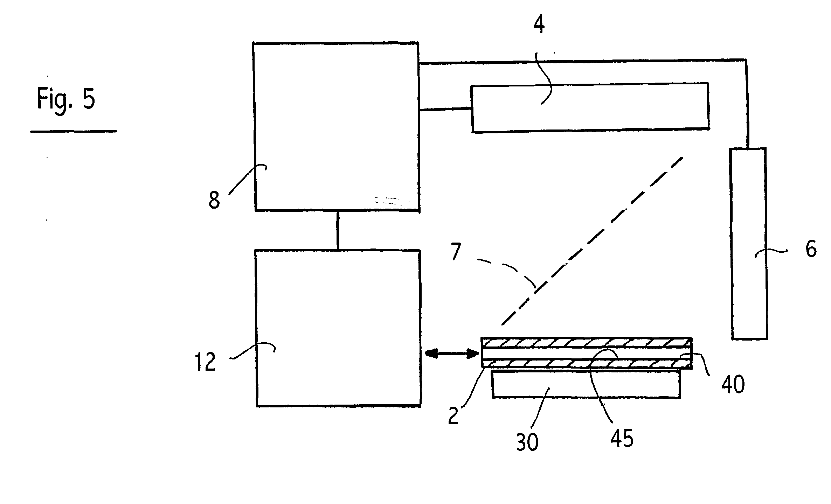

In Fig. 5 ist ein Blockschaltbild einer erfindungsgemäßen Vorrichtung für Auflichtbeobachtung gezeigt. Entsprechend den Bezugszeichen in Fig. 1 umfasst die Vorrichtung einen Steuerrechner 8, eine Belichtungsmatrix 4, eine Detektionsmatrix 6, ein Fluid-Handlingsystem (Fluidmodul) 12 für die Zufuhr der Beadsuspension zum Trägerkörper 2 und für die Zufuhr der für die Rezeptorsynthese erforderlichen Chemikalien. Als Einrichtung 30 zur Immobilisierung der Mikrobeads am Trägerkörper 2 ist eine ggf. wahlweise aus der dargestellten Position entfernbare Magnetvorrichtung an der der Belichtungsmatrix 4 abgewandten Seite des Trägerkörpers 2 vorgesehen. Die Einrichtung 30 kann Magnetspulen oder/und Permanentmagneten enthalten. Mit 7 ist ein optischer Strahlteiler in Fig. 5 gekennzeichnet, der Licht der Belichtungsmatrix 4 zum Trägerkörper 2 hin durchlässt. Vom Trägerkörper 2 kommendes Licht reflektiert der Strahlteiler 7 zur Detektionsmatrix 6. Bedarfsweise kann der Strahlteiler 7 aus dem Strahlengang zwischen Belichtungsmatrix 4 und Trägerkörper 2 herausbewegt werden.

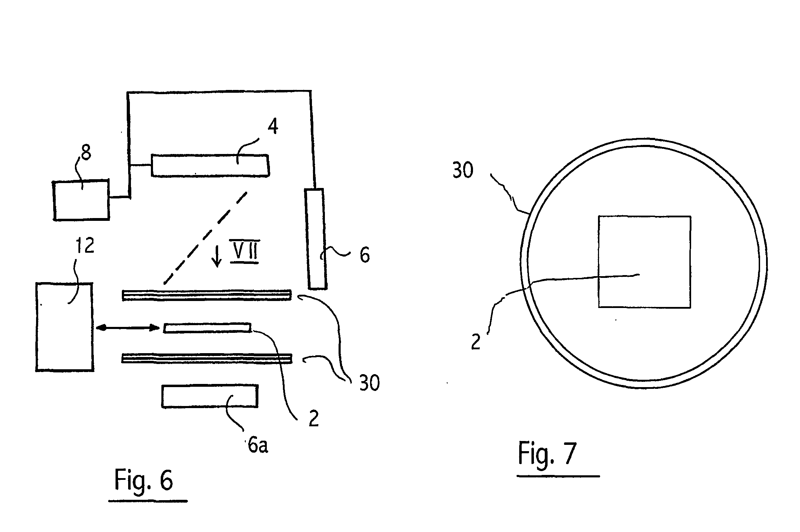

Bei der in Fig. 6 schematisch dargestellten Variante der Vorrichtung aus Fig. 5 weist die Einrichtung zur Immobilisierung der Beads ein Spulenpaar 30, z.B. Helmholtz-Spulenpaar, zur Erzeugung eines B-Feldes auf. Eine Spule des Spulenpaares befindet sich unterhalb des Trägerkörpers 2, wohingegen die andere Spule oberhalb des Trägers 2 positioniert ist. Das Spulenpaar 30 kann so gestaltet sein, dass es ein inhomogenes B-Feld erzeugt, um eine gerichtete Kraft auf die Pratikel auszuüben.

Fig. 7 zeigt eine Draufsicht auf den Trägerkörper 2 und die Spulen 30, wobei zu erkennen ist, dass die Spulen 30 den Trägerkörper 2 im Belichtungsstrahlengang nicht abschatten. Dies bedeutet auch, dass mittels der Detektionsmatrix 6a Durchlichtbeobachtung des Trägerkörpers 2 und der darin bzw. daran vorgesehenen Partikel durchgeführt werden kann.

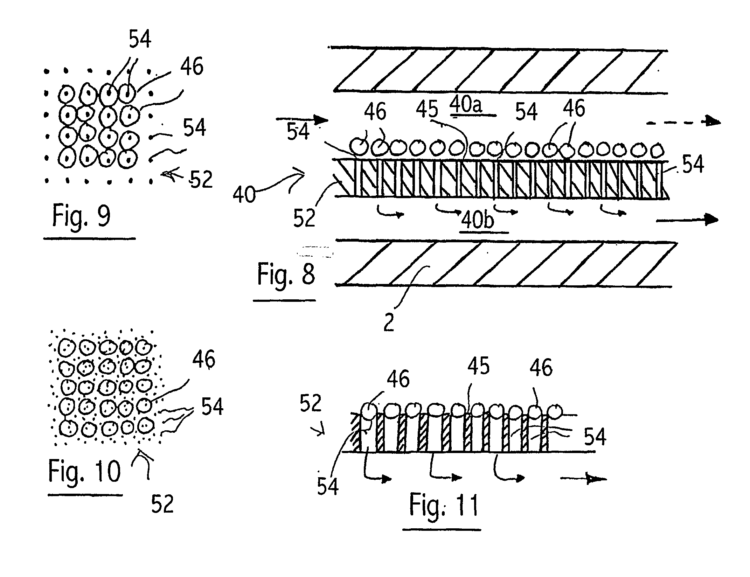

Bei den bisherigen Ausführungsbeispielen erfolgte die Immobilisierung der Mikrobeads mittels magnetischer, elektrischer bzw. elektromagnetischer Felder. Fig. 8 illustriert eine andere Möglichkeit der Immobilisierung von Mikrobeads in einem Mikrokanal 40 des Trägerkörpers 2. Fig. 8 zeigt ausschnittsweise einen Längsschnitt durch einen Fluidkanal 40, der von einer Zwischenwand 52 in einen Hauptströmungskanal 40a und einen Unterdruckkanal 40b unterteilt ist. Die Zwischenwand 52 weist eine Vielzahl von Mikroporen 54 auf, welche den Hauptströmungskanal 40a mit dem Unterdruckkanal 40b verbinden. Der Unterdruckkanal 40b ist an einer einen Unterdruck gegenüber dem Druck im Hauptströmungskanl 40a erzeugenden Pumpe angeschlossen. Die Mikrobeads 46 im Hauptströmungskanal 40a werden bei der Anordnung gemäß Fig. 8 an der Zwischenwand 52 aufgrund der eingestellten Druckverhältnisse immobilisiert. Der Durchmesser der Mikroporen 54 kann sehr viel kleiner sein als der Durchmesser der Mikrobeads, wie es in Fig. 8 angedeutet ist.

Fig. 9 zeigt eine schematische Draufsicht auf einen Bereich der Zwischenwand 52 in Fig. 8. In Fig. 9 ist zu erkennen, dass die Poren 54 geordnet in einem Raster vorgesehen sind, so dass eine entsprechende Rasteranordnung der immobilisierten Beads 46 erfolgen kann.

Alternativ könnte eine statistische bzw. zufällige Verteilung der Poren mit großer Porendichte in einer Zwischenwand 52 vorgesehen sein, wie dies in einer der Perspektive gemäß Fig. 9 entsprechenden Ansicht in Fig. 10 angedeutet ist. In diesem Sinne könnte die Zwischenwand 52 aus einem in sich porösen Material bestehen.

Aus der Detaildarstellung einer Zwischenwand in Fig. 11 ist zu erkennen, dass eine Variante möglich ist, bei der der Durchmesser der Poren 54 nur geringfügig kleiner ist als der Durchmesser der Mikrobeads 46.

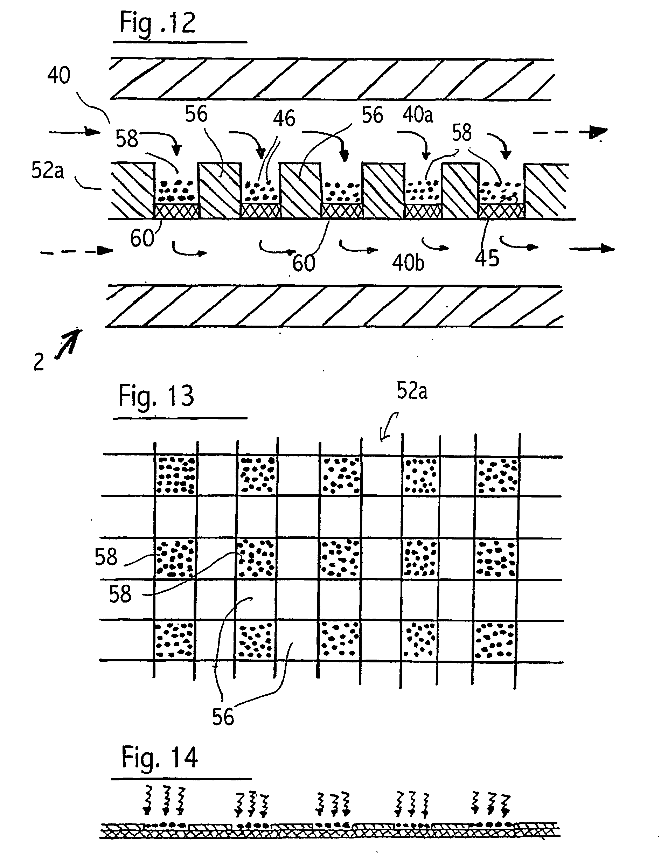

Fig. 12 zeigt eine weitere Möglichkeit der Immobilisierung von Mikrobeads 46 an einem Trägerkörper 2 auf. Fig. 12 zeigt ausschnittsweise einen Längsschnitt durch einen Fluidkanal 40 des nun betrachteten Trägerkörpers 2. In dem Fluidkanal 40 ist eine Zwischenwand 52a vorgesehen, welche den Fluidkanal in einen oberen Hauptströmungskanal 40a und einen unteren Unterdruckkanal 40b unterteilt. Im Unterdruckkanal 40b herrscht geringerer Druck als im Hauptströmungskanal 40a.

Die Zwischenwand 52a weist zwischen Mikrosäulen 56 aus Silizium, Foturan oder Kunststoff etc. Vertiefungen 58 auf, deren Böden 60 Mikroporen enthalten oder aus in sich porösem Material bestehen. Die Mikroporen in den Böden 60 erlauben Fluidströmung vom Hauptströmungskanal 40a zum Unterdruckkanal 40b mit entsprechendem Druckabfall. Dabei kommt es zur Anlagerung von in einer Suspension zugeführten Mikrobeads 46 in den Vertiefungen 58.

Wie die ausschnittsweise Draufsicht auf die Zwischenwand 52a gemäß Fig. 13 erkennen lässt, sind die Vertiefungen 58 und Säulen 56 im Beispielsfall nach Art eines Schachbrett-Musters angeordnet.

In Fig. 14 ist illustriert, dass die Vertiefungen 58 alternativ so gestaltet sein können, dass lediglich eine Lage an Mikrobeads 46 jeweils aufgenommen werden kann.

Es sei darauf hingewiesen, dass bei den Ausführungsformen nach den Figuren 8 - 14 unterstützend ein B-Feld oder/und ein E-Feld bei entsprechend magnetisch bzw. elektrisch präparierten Mikrobeads zu deren Immobilisierung verwendet werden kann.

Es sei ferner darauf hingewiesen, dass Mittel zur ganzheitlichen oder lokalen Temperierung des Trägers durch optischen oder elektrischen Energieeintrag vorgesehen sein können. Denkbar sind mikrotechnisch in den Trägerkörper integrierte Widerstandsheizelemente, die ortsspezifische Temperierung ermöglichen.

Weiterhin ist allgemein darauf hinzuweisen, dass bei Ausführungsformen mit elektrischer Immobilisierung der Beads ein den betreffenden Trägerkörper 2 ganzheitlich durchsetzendes E-Feld oder ortsspezifisch lokale elektrische Felder Anwendung finden können, wobei letztere z.B. durch individuell angesteuerte Elektroden auf einer CMOS-Schaltung und/oder mittels transparenter LCD-Elektroden erzeugt werden können.

Auch bei den Ausführungsformen mit magnetischer Immobilisierung der Beads kann es vorgesehen sein, dass ein den Trägerkörper ganzheitlich durchsetztes Magnetfeld erzeugt wird oder dass alternativ dazu ortsaufgelöst lokale Magnetfelder durch Spulen oder Permanentmagnete erzeugt werden. Denkbar sind in den Trägerkörper integrierte Mikrospulen zur Erzeugung lokaler Magnetfelder.

Die Beads können ggf. transparent oder zumindest teiltransparent sein.

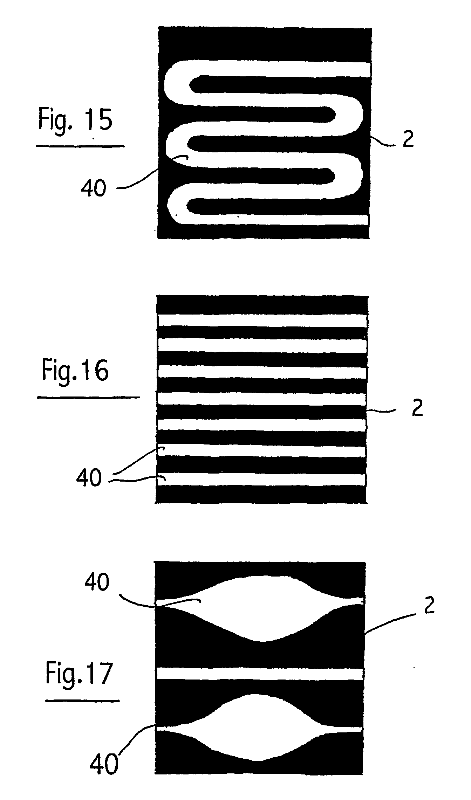

Die Figuren 15 - 17 zeigen ausschnittsweise in Vergrößerung Draufsichten auf verschiedene Varianten der Mikrofluidkanalgestaltung. Die Begrenzungsflächen der Mikrokanäle weisen vorzugsweise inerte Oberflächen auf.

## Patentansprüche

1. Verfahren zur integrierten Synthese und Analytbestimmung an einem Träger umfassend die Schritte:
a) Bereitstellen eines Trägerkörpers (2),
b) Leiten einer Flüssigkeit mit Partikeln (46) in oder auf den Trägerkörper (2),
c) Immobilisieren der Partikel (46) auf mindestens einer inneren oder/und äußeren Oberfläche (45) des Trägerkörpers (2),
d) Leiten einer Flüssigkeit mit darin enthaltenen Rezeptoren oder Bausteinen (A, B) für die Synthese von polymeren Rezeptoren über die immobilisierten Partikel (46),
e) orts- oder/und zeitspezifisches Koppeln der Rezeptoren oder Rezeptorbausteine (A, B) an jeweils vorbestimmten Positionen des Trägerkörpers (2) an die immobilisierten Partikel (46),
f) gegebenenfalls Wiederholen der Schritte (d) und (e), bis die gewünschten Rezeptoren an den jeweils vorbestimmten Positionen des Trägerkörpers auf den immobilisierten Partikeln (46) synthetisiert worden sind,
g) Inkontaktbringen der Rezeptoren mit einer den oder die zu bestimmenden Analyten enthaltenden Probe, und
h) Bestimmen des oder der Analyten über eine Bindung an die Rezeptoren, die an die immobilisierten Partikel (46) gekoppelt sind.

2. Verfahren nach Anspruch 1, **dadurch gekennzeichnet, dass** Schritt (c) ein permanentes Immobilisieren umfasst.

3. Verfahren nach Anspruch 2, **dadurch gekennzeichnet, dass** das permanente Immobilisieren eine kovalente chemische Bindung umfasst.

4. Verfahren nach Anspruch 1, **dadurch gekennzeichnet, dass** Schritt (c) ein nicht permanentes Immobilisieren umfasst.

5. Verfahren nach Anspruch 4, **dadurch gekennzeichnet, dass** das nicht permanente Immobilisieren eine magnetische, elektrische oder/und mechanische Wechselwirkung umfasst.

6. Verfahren nach Anspruch 4 oder 5, weiterhin umfassend das Ablösen der immobilisierten Partikel (46) von der Trägeroberfläche (45) und das Leiten der Partikel (46) aus dem Trägerkörper (2).

7. Verfahren nach einem der vorstehenden Ansprüche, **dadurch gekennzeichnet, dass** die Synthese und Analytbestimmung in einer integrierten Vorrichtung durchgeführt wird, wobei der Syntheseoder/und der Analytbestimmungsprozess in einer beliebigen Anzahl von Positionen auf dem Träger überwacht und geregelt wird.

8. Verfahren nach Anspruch 7, **dadurch gekennzeichnet, dass** man eine integrierte Vorrichtung verwendet, umfassend eine programmierbare Lichtquellenmatrix (4), eine Detektormatrix (6), einen vorzugsweise zwischen Lichtquellen- und Detektormatrix angeordneten zumindest bereichsweise transparenten Trägerkörper (2) sowie Mittel zur Zufuhr von Fluids in den Trägerkörper und zur Ableitung von Fluids aus dem Trägerkörper.

9. Verfahren nach einem der vorstehenden Ansprüche, **dadurch gekennzeichnet, dass** nach der Bestimmung der Analyt wieder vom Trägerkörper entfernt wird.

10. Verfahren nach einem der vorstehenden Ansprüche, **dadurch gekennzeichnet, dass** mehrere Synthese/Analytbestimmungszyklen durchgeführt werden, wobei die Rezeptoren für einen nachfolgenden Zyklus auf Basis der Informationen aus einem vorhergehenden Zyklus synthetisiert werden.

11. Verfahren nach Anspruch 10, **dadurch gekennzeichnet, dass** für den nachfolgenden Zyklus eine Verlängerung der Rezeptoren aus dem vorhergehenden Zyklus erfolgt.

12. Verfahren nach Anspruch 11, **dadurch gekennzeichnet, dass** für den nachfolgenden Zyklus ein neuer Trägerkörper mit gegenüber dem vorhergehenden Zyklus modifizierten Rezeptoren synthetisiert wird.

13. Verfahren nach Anspruch 12, **dadurch gekennzeichnet, dass** die Modifizierung der Rezeptoren eine Änderung der Sequenz oder/und einen Ausschluss negativer Rezeptoren des vorhergehenden Zyklus umfasst.

14. Verfahren nach einem der vorstehenden Ansprüche, **dadurch gekennzeichnet, dass** man einen Trägerkörper (2) mit einer Vielzahl von Kanälen (40) verwendet.

15. Verfahren nach einem der vorstehenden Ansprüche, **dadurch gekennzeichnet, dass** für einen Synthese/Analytbestimmungszyklus mehrere Trägerkörper (2) verwendet werden.

16. Verfahren zum Bestimmen eines oder mehrerer Analyten auf einem Träger umfassend die Schritte:
a) Bereitstellen eines Trägerkörpers (2) mit mindestens einer inneren oder/und äußeren Oberfläche (45), wobei Partikel (46) mit daran gekoppelten Rezeptoren an der Oberfläche (45) immobilisiert sind, wobei mehrere kodierte Partikelspezies mit jeweils unterschiedlichen Rezeptoren verwendet werden und die Immobilisierung eine magnetische oder/und elektrische Wechselwirkung umfasst,
g) Inkontaktbringen des Trägerkörpers bzw. der Rezeptoren mit einer den oder die zu bestimmenden Analyten enthaltenden Probe, und
h) Bestimmen des oder der Analyten über eine Bindung an die Rezeptoren, die an die immobilisierten Partikel (46) gekoppelt sind.

17. Vorrichtung zur Durchführung des Verfahrens nach einem der Ansprüche 1 - 15 oder zur Durchführung des Verfahrens nach Anspruch 16, umfassend
einen Trägerkörper (2), welcher wenigstens einen inneren oder/und einen äußeren Oberflächenbereich (45) zur Anlagerung von an ihrer Oberfläche funktionalisierten Partikeln (46), insbesondere Mikrobeads, aufweist,
Mittel zur Zuführung der Partikel (46) zu dem wenigstens einen Oberflächenbereich (45) des Trägerkörpers (2),
eine Einrichtung (30) zur Immobilisierung der Partikel (46) an den wenigstens einen Oberflächenbereich (45) des Trägerkörpers (2), die dazu eingerichtet ist, die Partikel (46) vermittels einer magnetischen - oder/und elektrischen - oder/und mechanischen Wechselwirkung an dem Oberflächenbereich (45) zu immobilisieren,
Mittel zur Zuführung von Rezeptoren oder Rezeptorbausteinen A, B zu auf dem wenigstens einen Oberflächenbereich (45) immobilisierten Partikeln (46) und
Mittel (4, 6, 8) zur Steuerung des orts- und/oder zeitspezifischen Koppelns der Rezeptoren oder Rezeptorbausteine A, B an die immobilisierten Partikel (46) an jeweils vorbestimmten Positionen des Trägerkörpers (2).

18. Vorrichtung nach Anspruch 17, wobei der Trägerkörper (2) innere oder/und äußere Kanäle, insbesondere Mikrokanäle (40) hat, welche Oberflächenbereiche (45) für die Immobilisierung der Partikel (46) aufweisen.

19. Vorrichtung nach Anspruch 18, wobei die Partikel (46) magnetisch sind und wobei die Einrichtung (30) zur Immobilisierung der Partikel (46) Mittel zur Erzeugung eines den Trägerkörper (2) durchsetzenden magnetischen Feldes B aufweisen.

20. Vorrichtung nach Anspruch 19, wobei die Magnetfelderzeugungsmittel (30) elektromagnetische Spulen, insbesondere wenigstens ein Helmholtz-Spulenpaar, oder/und wenigstens einen Permanentmagneten umfassen.

21. Vorrichtung nach einem der Ansprüche 17 - 20, wobei die Partikel (46) elektrisch geladen bzw. elektrisch polarisiert sind und wobei die Einrichtung (30) zur Immobilisierung der Partikel (46) Mittel zur Erzeugung eines den Trägerkörper (2) durchsetzenden elektrischen Feldes E umfassen.

22. Vorrichtung nach einem der Ansprüche 17 - 21, wobei die Einrichtung (30) zur Immobilisierung der Partikel (46) Porenöffnungen in dem wenigstens einen Trägerkörper-Oberflächenbereich (45) für die Immobilisierung der Partikel (46) und Mittel zur Erzeugung einer die betreffenden Partikel (46) an dem Oberflächenbereich (45') haltenden Saugwirkung an den Porenöffnungen (47) umfasst.

23. Vorrichtung nach Anspruch 22, wobei die mit Porenöffnungen (47) versehenen Oberflächenbereiche in Vertiefungen (58) von Mikrofluidkanälen (40a) angeordnet sind.

24. Vorrichtung nach Anspruch 22 oder 23, wobei die Porenöffnungsdurchmesser kleiner als 90 % des kleinsten Partikeldurchmessers, vorzugsweise kleiner als 30 % des kleinsten Partikeldurchmessers sind.

25. Vorrichtung nach einem der Ansprüche 17 - 24, wobei die Einrichtung (30) zur Immobilisierung der Partikel (46) dazu eingerichtet ist, gesteuert ortsspezifisch und ortsaufgelöst Haltekräfte in wahlweise unterschiedlichen Abschnitten der Oberflächenbereiche (46) des Trägerkörpers (2) auf betreffende Partikel auszuüben.

26. Vorrichtung nach einem der Ansprüche 17 - 25, wobei die Mittel zur Steuerung der orts- oder/und zeitspezifischen Kopplung der Rezeptoren oder Rezeptorbausteine (A, B) an die immobilisierten Partikel (46) eine Belichtungsmatrix (4) zur Erzeugung programmierbar steuerbarer Belichtungsmuster aufweist und wobei der Trägerkörper (2) wenigstens einseitig an der Seite der Belichtungsmatrix (4) zu dem wenigstens einen Oberflächenbereich (45) hin für Licht der Belichtungsmatrix (4) transparent ist.

27. Vorrichtung nach Anspruch 26, wobei sie ferner eine Lichtdetektionsmatrix (6) zur optischen Überwachung der Partikelimmobilisierung oder/und der Synthesevorgänge oder/und von Analytbestimmungsvorgängen im Trägerkörper (2) aufweist.

28. Vorrichtung nach einem der Ansprüche 17 - 27, wobei die Partikel (46) im Wesentlichen gleich große Durchmesser aufweisen und wobei der Partikeldurchmesser kleiner als 50 µm, insbesondere kleiner als 10 µm, ist.

## Claims

1. A method for integrated synthesis and analyte determination on a support, comprising the following steps:
a) providing a support body (2) ,
b) conducting a liquid containing particles (46) into or onto the support body (2),
c) immobilising the particles (46) on at least one inner or/and outer surface (45) of the support body (2) ,
d) conducting a liquid which contains receptors or building blocks (A, B) for the synthesis of polymer receptors over the immobilised particles (46),
e) coupling the receptors or receptor building blocks (A, B) location-specifically or/and time-specifically to the immobilised particles (46) at in each case predetermined positions of the support body (2),
f) where appropriate, repeating steps (d) and (e), until the desired receptors have been synthesised on the immobilised particles (46) at the in each case predetermined positions of the support body,
g) bringing the receptors into contact with a sample containing the analyte(s) to be determined, and
h) determining the analyte(s) via binding to the receptors which are coupled to the immobilised particles (46).

2. A method according to Claim 1,
**characterised in that** step (c) comprises permanent immobilisation.

3. A method according to Claim 2,
**characterised in that** permanent immobilisation comprises a covalent chemical bond.

4. A method according to Claim 1,
**characterised in that** step (c) comprises non-permanent immobilisation.

5. A method according to Claim 4,
**characterised in that** non-permanent immobilisation comprises a magnetic, electrical and/or mechanical interaction.

6. A method according to Claim 4 or 5, furthermore comprising the detachment of the immobilised particles (46) from the support surface (45) and the conducting of the particles (46) out of the support body (2).

7. A method according to one of the preceding Claims,
**characterised in that** the synthesis and analyte determination are carried out in an integrated apparatus, the synthesis process or/and analyte determination process being monitored and controlled in any number of positions on the support.

8. A method according to Claim 7,
**characterised in that** an integrated apparatus is used, which comprises a programmable light source matrix (4), a detector matrix (6), a support body (2) which is preferably arranged between the light source matrix and data matrix and has at least some transparent zones, and means for supplying fluid into the support body and for discharging fluid from the support body.

9. A method according to one of the preceding Claims,
**characterised in that** the analyte is removed from the support body again after the determination.

10. A method according to one of the preceding Claims,
**characterised in that** several synthesis/analyte determination cycles are carried out, the receptors being synthesised for a subsequent cycle on the basis of the information from a preceding cycle.

11. A method according to Claim 10,
**characterised in that** the receptors from the preceding cycle are extended for the subsequent cycle.

12. A method according to Claim 11,
**characterised in that** a new support body having modified receptors compared with the preceding cycle is synthesised for the subsequent cycle.

13. A method according to Claim 12,
**characterised in that** the modification of the receptors comprises changing the sequence or/and eliminating negative receptors of the preceding cycle.

14. A method according to one of the preceding Claims,
**characterised in that** a support body (2) having a plurality of channels (40) is used.

15. A method according to one of the preceding Claims,
**characterised in that** several support bodies (2) are used for a synthesis/analyte determination cycle.

16. A method for determining one or more analytes on a support, comprising the following steps:
a) providing a support body (2) with at least one inner or/and outer surface (45), in which particles (46) with receptors coupled thereto are immobilised to the surface (45), with several coded particle species with in each case different receptors being used, and the immobilisation comprising a magnetic or/and electrical interaction,
g) bringing the support body or the receptors into contact with a sample containing the analyte or analytes to be determined, and
h) determining the analyte or analytes via binding to the receptors coupled to the immobilised particles (46).

17. An apparatus for performing the method according to one of Claims 1 - 15 or performing the method according to Claim 16, comprising:
- a support body (2), which comprises at least one inner or/and one outer surface region (45) for attaching particles (46), in particular microbeads, whose surface has been functionalised,
- means for supplying the particles (46) to the at least one surface region (45) of the support body (2),
- a device (30) for immobilising the particles (46) on the at least one surface region (45) of the support body (2), which is set up to immobilise the particles (46) on the surface region (45) by means of a magnetic - or/and electrical - or/and mechanical interaction,
- means for supplying receptors or receptor building blocks A, B to particles (46) immobilised on the at least one surface region (45),
- means (4, 6, 8) for controlling location-specific and/or time-specific coupling of the receptors or receptor building blocks A, B to the immobilised particles (46) at in each case predetermined positions of the support body (2).

18. An apparatus according to Claim 17, in which the support body (2) has inner or/and outer channels, in particular microchannels (40), which comprise surface regions (45) for immobilising the particles (46).

19. An apparatus according to Claim 18, in which the particles (46) are magnetic and in which the device (30) for immobilising the particles (46) have means for generating a magnetic field B pervading the support body (2).

20. An apparatus according to Claim 19, in which the magnetic field generating means (30) comprise electromagnetic coils, in particular at least one Helmholtz coil pair, or/and at least one permanent magnet.

21. An apparatus according to one of Claims 17 - 20, in which the particles (46) are electrically charged or electrically polarised and in which the device (30) for immobilising the particles (46) comprise means for generating an electric field E passing through the support body (2).

22. An apparatus according to one of Claims 17 - 21, in which the device (30) for immobilising the particles (46) comprise pore openings in the at least one support body surface region (45) for immobilising the particles (46) and means for generating a suction action at the pore openings (47), which holds the respective particles (46) at the surface region (45').

23. An apparatus according to Claim 22, in which the surface regions provided with pore openings (47) are arranged in depressions (58) of microfluid channels (40a).

24. An apparatus according to Claim 22 or 23, in which the diameters of the pore openings are less than 90 % of the smallest particle diameter, preferably less than 30 % of the smallest particle diameter.

25. An apparatus according to one of Claims 17 - 24, in which the device (30) for immobilising the particles (46) is set up for the purpose of exerting, in a controlled location-specific and space-resolved manner, holding forces on particles in question in optionally different sections of the surface regions (46) of the support body (2).

26. An apparatus according to one of Claims 17 - 25, in which the means for controlling location or/and time-specific coupling of the receptors or receptor building blocks (A, B) to the immobilised particles (46) comprises an illumination matrix (4) for generating illumination patterns which can be controlled in a programmable manner, and in which apparatus the support body (2) is, at least on one side, transparent for light from the illumination matrix (4) on the side of the illumination matrix facing the at least one surface region (45).

27. An apparatus according to Claim 26, which also has a light detection matrix (6) for optically monitoring the particle immobilisation or/and the synthesis processes or/and analyte determination processes in the support body (2).

28. An apparatus according to one of Claims 17 - 27, in which the particles (46) have diameters of essentially the same size and in which the particle diameter is less than 50 µm, in particular less than 10 µm.

## Revendications

1. Procédé de synthèse intégrée et de détection d'analytes sur un support, comprenant les étapes :
a) préparation d'un corps support (2),
b) passage d'un liquide avec des particules (46) dans ou sur le corps support (2),
c) immobilisation des particules (46) sur au moins une surface interne et/ou externe (45) du corps support (2),
d) passage d'un liquide contenant les récepteurs ou éléments (A, B) pour la synthèse de récepteurs polymères sur les particules immobilisées (46),
e) couplage spécifique de site et/ou de temps des récepteurs ou des éléments de récepteur (A, B) sur une position chaque fois prédéterminée du corps support (2) sur les particules immobilisées (46),
f) le cas échéant, répétition des étapes (d) et (e), jusqu'à ce que les récepteurs souhaités aient été synthétisés en les positions chaque fois prédéterminées du corps support, sur les particules immobilisées (46),
g) mise en contact des récepteurs avec un échantillon contenant le ou les analytes à détecter, et
h) détection du ou des analytes par une liaison sur les récepteurs, qui sont couplés sur les particules immobilisées (46).

2. Procédé selon la revendication 1, **caractérisé en ce que** l'étape (c) comprend une immobilisation permanente.

3. Procédé selon la revendication 2, **caractérisé en ce que** l'immobilisation permanente comprend une liaison chimique covalente.

4. Procédé selon la revendication 1, **caractérisé en ce que** l'étape (c) comprend une immobilisation non permanente.

5. Procédé selon la revendication 4, **caractérisé en ce que** l'immobilisation non permanente comprend une interaction magnétique, électrique et/ou mécanique.

6. Procédé selon la revendication 4 ou 5, comprenant d'autre part, la séparation des particules immobilisées (46) de la surface support (45) et l'évacuation des particules (46) du corps support (2).

7. Procédé selon l'une des revendications précédentes, **caractérisé en ce que** la synthèse et la détection d'analyte sont réalisées dans un dispositif intégré, où le processus de synthèse et/ou de détection d'analyte est surveillé et réglé en un nombre quelconque de positions sur le support.

8. Procédé selon la revendication 7, **caractérisé en ce que** l'on utilise un dispositif intégré, comprenant une matrice source de lumière (4) programmable, une matrice détecteur (6), un corps support (2) au moins partiellement transparent, de préférence disposé entre la matrice source de lumière et la matrice détecteur, ainsi qu'un moyen pour amener les fluides dans le corps support et évacuer les fluides du corps support.

9. Procédé selon l'une des revendications précédentes, **caractérisé en ce qu'**après la détection, l'analyte est éliminé du corps support.

10. Procédé selon l'une des revendications précédentes, **caractérisé en ce que** plusieurs cycles synthèse/détection d'analyte sont réalisés, où les récepteurs sont synthétisés lors d'un cycle suivant, à partir des informations provenant d'un cycle précédent.

11. Procédé selon la revendication 10, **caractérisé en ce que** lors du cycle suivant, on réalise un allongement du récepteur provenant du cycle précédent.

12. Procédé selon la revendication 11, **caractérisé en ce que** lors du cycle suivant, on synthétise un nouveau corps support avec le récepteur modifié par rapport au cycle précédent.

13. Procédé selon la revendication 12, **caractérisé en ce que** la modification du récepteur comprend un changement de séquence et/ou une élimination des récepteurs négatifs du cycle précédent.

14. Procédé selon l'une des revendications précédentes, **caractérisé en ce que** l'on utilise un corps support (2) avec une pluralité de canaux (40).

15. Procédé selon l'une des revendications précédentes, **caractérisé en ce que** l'on utilise, pour un cycle de synthèse/détection d'analyte, plusieurs corps support (2).

16. Procédé de détection d'un ou de plusieurs analytes sur un support, comprenant les étapes de :
a) préparation d'un corps support (2), avec au moins une surface interne et/ou une surface externe (45), où des particules (46) avec récepteurs y couplés sont immobilisées sur la surface (45), où plusieurs espèces codées de particules avec chaque fois, des récepteurs différents, sont utilisées et l'immobilisation comprend une interaction magnétique et/ou électrique,
g) mise en contact du corps support ou des récepteurs avec un échantillon contenant le ou les analytes à détecter, et
h) détection du ou des analytes par une liaison sur les récepteurs, qui sont couplés sur les particules immobilisées (46).

17. Dispositif pour réaliser le procédé selon l'une des revendications 1-15 ou pour réaliser le procédé selon la revendication 16, comprenant
un corps support (2), qui présente au moins une surface interne et/ou une surface externe (45), pour la fixation de particules (46) fonctionnalisées en surface, en particulier de microbilles,
un moyen pour amener les particules (46) à la au moins une zone de surface (45) du corps support (2),
un dispositif (30) pour immobiliser les particules (46) sur la au moins une surface (45) du corps support (2), qui est équipé de sorte que les particules (46) sont immobilisées par une interaction magnétique et/ou électrique et/ou mécanique sur la zone de surface (45),
un moyen pour amener des récepteurs ou éléments de récepteur A, B sur les particules (46) immobilisées sur la au moins une zone de surface (45), et
des moyens (4, 6, 8) pour régler le couplage spécifique de site et/ou de temps des récepteurs ou éléments de récepteur A, B sur les particules immobilisées (46) en chaque position prédéterminée du corps support (2).

18. Dispositif selon la revendication 17, où le corps support (2) a des canaux internes et/ou externes, en particulier des microcanaux (40), qui présentent des zones de surface (45) pour l'immobilisation des particules (46).

19. Dispositif selon la revendication 18, où les particules (46) sont magnétiques et où le dispositif (30) pour l'immobilisation des particules (46) présente des moyens pour produire un champ magnétique B traversant le corps support (2).

20. Dispositif selon la revendication 19, où les moyens de production de champ magnétique (30) comprennent des bobines électromagnétiques, en particulier au moins une paire de bobine de Helmholtz, et/ou au moins un aimant permanent.

21. Dispositif selon l'une des revendications 17-20, où les particules (46) sont chargées électriquement ou polarisées électriquement et où le dispositif (30) pour l'immobilisation des particules (46) comprend des moyens pour produire un champ électrique E traversant le corps support (2).

22. Dispositif selon l'une des revendications 17-21, où le dispositif (30) pour l'immobilisation des particules (46) comprend des orifices de pore dans la au moins une zone de surface du corps support (45) pour l'immobilisation des particules (46) et des moyens pour produire une action d'aspiration maintenant les particules (46) sur la zone de surface (45') sur les orifices de pore (47).

23. Dispositif selon la revendication 22, où les zones de surface munies d'orifices de pore (47) sont disposées dans les approfondissements (58) de microcanaux fluides (40a).

24. Dispositif selon la revendication 22 ou 23, où le diamètre des orifices de pore est inférieur à 90% du diamètre le plus petit des particules, de préférence inférieur à 30% du diamètre le plus petit des particules.

25. Dispositif selon l'une des revendications 17-24, où le dispositif (30) pour immobiliser les particules (46) est équipé de manière à exercer sur les particules, une force de maintien ciblée, spécifique de site dans différentes sections choisies de la zone de surface (46) du corps support (2).

26. Dispositif selon l'une des revendications 17-25, où le moyen pour régler le couplage spécifique de site et/ou de temps des récepteurs ou éléments de récepteur (A, B) sur les particules immobilisées (46), présente une matrice d'illumination (4) pour produire un modèle programmable, réglable d'illumination et où le corps support (2) est transparent au moins sur une face, sur la face de la matrice d'illumination (4), vers la au moins une zone de surface (45), pour la lumière de la matrice d'illumination.

27. Dispositif selon la revendication 26, où il présente en outre, une matrice détecteur de lumière (6) pour la surveillance optique de l'immobilisation des particules et/ou le processus de synthèse et/ou les processus de détection d'analyte dans le corps support (2).

28. Dispositif selon l'une des revendication 17-27, où les particules (46) présentent un diamètre essentiellement de même taille et où le diamètre des particules est inférieur à 50 µm, en particulier inférieur à 10 µm.
